# EUROPÄISCHE PATENTANMELDUNG

(11) **EP 3 454 342 A1**
(43) Veröffentlichungstag der Anmeldung: **13.03.2019**
(21) Anmeldenummer: 17190562.3
(22) Anmeldetag: 12.09.2017
(51) Int. Cl.: G16H 50/20, G16H 10/60, G16H 30/40

(54) **AUTOMATISIERTES ERMITTELN UND VERARBEITEN VON PATIENTENDATEN ZUR GESUNDHEITSBETREUUNG**

(71) Anmelder: Siemens Healthcare GmbH, 91052 Erlangen (DE)
(72) Erfinder: Ionasec, Razvan, 90402 Nuernberg (DE); Schmidt, Sebastian, 91085 Weisendorf (DE); Shah, Amitkumar Bhupendrakumar, 91052 Erlangen (DE)

(57) **Zusammenfassung**

Es wird ein Gesundheitsförderungssystem (1) beschrieben. Das Gesundheitsförderungssystem weist eine Auswertungseinrichtung (3a) auf, welche dazu eingerichtet ist, aus medizinischen Bilddaten (BD) von Patienten Merkmale hinsichtlich medizinischer Indikationen zu extrahieren und den medizinischen Bilddaten (BD) zugeordnete Medikamente und/oder Behandlungsverfahren zu ermitteln und auf Basis der extrahierten Merkmale und der ermittelten Medikamente und/oder Behandlungsverfahren kombinierte medizinische Daten (KMD) zu bilden. Zudem umfasst das Gesundheitsförderungssystem (1) eine zentrale Datenbank (4), welche dazu eingerichtet ist, die kombinierten medizinischen Daten (KMD) von der Auswertungseinrichtung (3a) zu empfangen und zu speichern. Teil des Gesundheitsförderungssystems ist auch eine Patientenbetreuungseinrichtung (5), welche dazu eingerichtet ist, für einen neuen Patienten automatisiert Informationen (IG, ISB) bezüglich seines Gesundheitszustands und spezifischer Behandlungsmöglichkeiten auf Basis eines Vergleichs zwischen dem neuen Patienten zugeordneten medizinischen Bilddaten (BD) und den in der zentralen Datenbank (4) abgespeicherten kombinierten medizinischen Daten (KMD) zu ermitteln. Es wird auch ein Gesundheitsbetreuungsverfahren beschrieben.

## Beschreibung

Die Erfindung betrifft ein Gesundheitsförderungssystem. Weiterhin betrifft die Erfindung ein Gesundheitsbetreuungsverfahren.

Die medizinische Bildgebung ist eine der wichtigsten Untersuchungstechniken, welche heutzutage genutzt werden, um unschätzbar wertvolle Informationen über den Gesundheitszustand von Personen und insbesondere Patienten zu erhalten. Es wurden viele verschiedene Modalitäten entwickelt, um unterschiedliche physiologische Aspekte erkennen zu können. Medizinische Bildgebungsverfahren haben weltweit Anwendung bei vielen medizinischen Problemen gefunden. Allerdings wird die gewonnene Bildinformation meist isoliert verwendet, nur um eine bestimmte medizinische Fragestellung zu adressieren, und dieser Vorgang erfolgt meistens entkoppelt von der folgenden medizinischen Behandlung oder Empfehlung hinsichtlich einer pharmakologischen Behandlung oder der Nutzung von Nahrungsergänzungsmitteln.

Personen, die sich einer Untersuchung unter Anwendung einer medizinischen Bildgebung unterziehen, könnten erheblich von zusätzlicher Information und zusätzlichen Empfehlungen profitieren, die sie anhand der erfassten Bilddaten über die primären diagnostischen Daten hinaus erhalten würden. Beispielsweise können Abbildungen des Körpers, wie zum Beispiel CT-Abbildungen oder MRT-Abbildungen des Magen-Darm-trakts, spezielle Informationen bezüglich des Körpergewichts und der Fettverteilung und des Fettanteils liefern und dazu könnten gleich Empfehlungen hinsichtlich einer pharmakologischen Gewichtsreduktion und einer Behandlung zur Senkung von Cholesterinwerten gegeben werden. Auf ähnliche Weise können Informationen hinsichtlich der Knochendichte und hinsichtlich Kalzifizierungen der Gefäße mit der Empfehlung von sinnvoll anwendbaren Medikamenten verknüpft werden.

Gleichzeitig kann die in den medizinischen Bildern enthaltene Information wertvolle Einsichten bezüglich der Inzidenz und Prävalenz bestimmter Gesundheitszustände in der Bevölkerung oder hinsichtlich des Effekts bestimmter verschreibungspflichtiger Medikamente oder freier Medikamente in einem bestimmten Bevölkerungsteil liefern. Beispielsweise kann die Verteilung von übergewichtigen Personen in bestimmten Regionen ermittelt werden, was der Pharmaindustrie gezielte Marketing- und Verteilungskampagnen erlauben würde. Außerdem könnten große Populationen mit ähnlichem Gesundheitszustand, welche mit verschiedenen Medikamenten behandelt werden, verglichen werden und die Information könnte dazu verwendet werden, Indikationen für den Gebrauch, die Dosierung und die Entwicklung neuer Präparate zu verbessern.

Bisher wird die medizinische Bildgebung für diagnostische Prozesse und für das Überwachen von minimal invasiven Therapievorgängen eingesetzt. Obwohl die Bilddaten in den verfügbaren Informationssystemen gespeichert und archiviert werden, werden die Daten fast nur dazu verwendet, um Informationen für die aktuelle primäre Indikation zu gewinnen. Mithin werden Informationen für andere Zwecke nicht aus den Bilddatengewonnen und auch nicht weiterverwendet. Außerdem werden Bilddaten fast ausschließlich in Verbindung mit anderen Informationen bezüglich eines bestimmten Patienten verwendet, ohne dass diese Daten innerhalb eines Volksgesundheitsverwaltungssystems (im englischen "population health management system") genutzt werden. Ein derartiges Volksgesundheitsverwaltungssystems (im englischen "population health management system") kann beispielsweise die Verwaltung bzw. optimierte Nutzung von Daten für eine bestimmte Population von Individuen, z.B. ein Volk, eine bestimmte Bevölkerungsgruppe oder ein bestimmtes Patientenkollektiv betreffen. Der Begriff "Volk" kann sich dabei auf die Staatsangehörigen eines bestimmten Staates oder einer Gruppe von Staaten (z.B. Mitgliedsstaaten der Europäischen Union), auf die Mitglieder einer bestimmten ethnischen Gruppe oder auf die Mitglieder eines bestimmten Gesundheitssystems beziehen. Der Begriff "Bevölkerungsgruppe" kann sich dabei beispielsweise auf eine Untergruppe eines "Volkes" beziehen, z.B. Personen oberhalb oder unterhalb eines bestimmten Alters, Personen eines bestimmten Geschlechts, o.ä.). Der Begriff "Patientenkollektiv" kann sich dabei auf eine Gruppe von Individuen mit einer bestimmten Krankheitsdiagnose beziehen, z.B. eine Gruppe von Diabetes-Patienten, eine Gruppe von Bluthochdruck-Patienten, o.ä.).

Daher wird komplementäre Information selten erhalten und verwendet, um einen bestimmten Patienten besser zu betreuen und ihm Informationen und Empfehlungen bezüglich des Nutzens bestimmter Medikamente zu geben. Solche Entscheidungen werden meistens auf Basis der Ermittlung von Symptomen gefällt, wodurch Möglichkeiten der Prävention eingeschränkt sind und die Behandlung des Patienten auf eine akute Krankheit beschränkt ist.

Bilddaten werden heutzutage nicht dahingehend ausgewertet, um Bevölkerungsstatistiken zu erstellen, mit Ausnahme speziell angelegter klinischer Studien, die sehr teuer sind, geringe Fallzahlen aufweisen und auf die jeweils analysierte Information beschränkt sind. Daher betreffen heute demographische Statistiken nur Parameter von allgemeiner Bedeutung mit sehr beschränktem Nutzen bezüglich einer gezielten Anwendung auf bestimmte Bevölkerungssegmente bezüglich der Anwendung von Pharmaprodukten.

Es besteht also das Problem, ein System und ein Verfahren zur Auswertung von medizinischen Bilddaten zu entwickeln, welches über die primäre Indikation der Patienten hinaus die Bildinformation für eine umfassende Betreuung des Patienten nutzt.

Diese Aufgabe wird durch ein Gesundheitsförderungssystem zum automatisierten Bearbeiten von Patientendaten gemäß Patentanspruch 1 und durch ein Gesundheitsbetreuungsverfahren gemäß Patentanspruch 12 gelöst.

Das erfindungsgemäße Gesundheitsförderungssystem weist eine Auswertungseinrichtung auf. Die Auswertungseinrichtung ist dazu eingerichtet, aus medizinischen Bilddaten von Patienten Merkmale hinsichtlich medizinischer Indikationen zu extrahieren. Die Auswertung der Bilddaten kann zum Beispiel das Ermitteln von Parametern, wie zum Beispiel Biomarker, also objektiv messbare biologische Merkmale, welche dazu genutzt werden können, den Gesundheitszustand einer Person oder deren Risikoprofil hinsichtlich zusätzlicher Erkrankungen einzuschätzen, umfassen. Weiterhin ist die Auswertungseinrichtung dazu eingerichtet, den medizinischen Bilddaten bzw. den jeweils extrahierten Merkmalen zugeordnete verschriebene bzw. angeforderte Medikamente und/oder Behandlungsverfahren zu ermitteln und auf Basis der extrahierten Merkmale, beispielsweise die genannten Biomarker, und der ermittelten Medikamente und/oder Behandlungsverfahren kombinierte medizinische Daten zu bilden. Der Begriff Behandlungsverfahren soll in diesem Zusammenhang sowohl ein Untersuchungsverfahren, wie zum Beispiel ein Screening-Verfahren im Rahmen einer Gesundheitsvorsorgemaßnahme, als auch ein Heilverfahren umfassen.

Teil des erfindungsgemäßen Gesundheitsförderungssystems ist auch eine zentrale Datenbank. Die zentrale Datenbank ist dazu eingerichtet, die kombinierten medizinischen Daten von der Auswertungseinrichtung zu empfangen und diese zu speichern.

Das erfindungsgemäße Gesundheitsförderungssystem umfasst zudem eine Patientenbetreuungseinrichtung, welche dazu eingerichtet ist, für einen neuen Patienten und/oder sein Betreuungspersonal automatisiert Informationen bezüglich seines Gesundheitszustands und spezifischer Behandlungsmöglichkeiten auf Basis eines Vergleichs zwischen dem neuen Patienten zugeordneten medizinischen Bilddaten und den in der zentralen Datenbank gespeicherten Daten zu ermitteln. Der Vergleich kann zum Beispiel einen Merkmalsvergleich zwischen extrahierten Merkmalen der aktuellen Bilddaten und entsprechenden Merkmalen von älteren, in der zentralen Datenbank abgespeicherten Daten umfassen. Ergibt sich bei dem Vergleich ein ähnliches Krankheitsbild, so können die im Zusammenhang mit den in der zentralen Datenbank abgespeicherten Informationen über bestellte Medikamente genutzt werden, um dem neuen Patienten ein entsprechendes Medikament oder ein geeignetes Behandlungsverfahren vorzuschlagen. Als "neuer" Patient soll ein Patient aufgefasst werden, für den in der Datenbank zu einer bestimmten medizinischen Fragestellung noch kein Datensatz bzw. kein aktueller Datensatz vorhanden ist.

Vorteilhaft werden mit Hilfe einer Datenbasis von ohnehin bei der Bildgebung und einer eventuellen Auswertung dieser Bilddaten anfallenden Daten, Informationen ermittelt, die dazu genutzt werden können, dem Patienten und/oder dem medizinischen Betreuungspersonal Hinweise hinsichtlich medizinischer Maßnahmen zu geben. Dabei soll die Patientenbetreuungseinrichtung keine Diagnose erstellen, sondern automatisiert Risikoprofile und/oder Eignungen von Patienten für bestimmte Medikamente, Untersuchungen, Vorsorgemaßnahmen oder Behandlungsmaßnahmen ermitteln.

Das Gesundheitsförderungssystem kann auch dazu genutzt werden, bereits hinsichtlich einer ersten Indikation ausgewertete Bilder hinsichtlich zusätzlicher Indikationen auszuwerten. Dabei werden Merkmale, welche mit diesen zusätzlichen Indikationen korrespondieren, aus dem bereits für die Prüfung auf die erste Indikation vorhandenen Bildmaterial extrahiert und mit entsprechenden kombinierten Daten aus der zentralen Datenbank abgeglichen.

Vorteilhaft werden zur Ermittlung dieser zusätzlichen Daten keine zusätzlichen Untersuchungen des Patienten benötigt, was den Aufwand verringert und die Akzeptanz und den Komfort für den Patienten verbessert. Anhand der vorliegenden Daten können zum Beispiel gewisse Risiken eines Patienten für bestimmte zusätzliche Erkrankungen ermittelt werden. Dem Patient kann dann die Teilnahme an einem geeigneten Überwachungsprogramm oder Vorsorgeprogramm empfohlen werden. Auf diese Weise können die genannten Überwachungsprogramme gezielt auf Patienten beschränkt werden, die ein erhöhtes Risiko für die betreffenden Krankheiten haben. Mithin kann die Zuverlässigkeit bzw. der positive Vorhersagewert solcher Überwachungsverfahren verbessert werden.

Bei dem erfindungsgemäßen Gesundheitsbetreuungsverfahren werden kombinierte medizinische Daten auf Basis von medizinischen Bilddaten von Patienten erzeugt. Aus den medizinischen Bilddaten werden Merkmale hinsichtlich medizinischer Indikationen extrahiert und den medizinischen Bilddaten zugeordnete bestellte Medikamente ermittelt. Auf Basis der extrahierten Merkmale und der medizinischen Bilddaten werden kombinierte medizinische Daten gebildet. Die kombinierten medizinischen Daten werden dann in einer zentralen Datenbank gespeichert. Weiterhin werden für einen neuen Patienten auf Basis eines Vergleichs zwischen dem neuen Patienten zugeordneten medizinischen Bilddaten und den in der zentralen Datenbank gespeicherten Daten automatisiert Informationen bezüglich des Gesundheitszustands und spezifischer Behandlungsmöglichkeiten eines Patienten ermittelt.

Das erfindungsgemäße Gesundheitsbetreuungsverfahren teilt die Vorteile des erfindungsgemäßen Gesundheitsförderungssystems.

Die abhängigen Ansprüche sowie die nachfolgende Beschreibung enthalten jeweils besonders vorteilhafte Ausgestaltungen und Weiterbildungen der Erfindung. Dabei können insbesondere die Ansprüche einer Anspruchskategorie auch analog zu den abhängigen Ansprüchen einer anderen Anspruchskategorie weitergebildet sein. Zudem können im Rahmen der Erfindung auch die verschiedenen Merkmale unterschiedlicher Ausführungsbeispiele und Ansprüche auch zu neuen Ausführungsbeispielen kombiniert werden.

In einer Ausgestaltung des erfindungsgemäßen Gesundheitsförderungssystems umfasst das erfindungsgemäße Gesundheitsförderungssystem zudem ein Volksgesundheitsverwaltungssystem, im englischen auch als "population health management system" bezeichnet, welches dazu eingerichtet ist, auf Basis der in der zentralen Datenbank gespeicherten Daten automatisiert statistische Daten betreffend den Gesundheitszustand von Patientenkollektiven und Statistiken über Behandlungsergebnisse zu ermitteln. Die erhaltenen Informationen können dazu genutzt werden, die Wirksamkeit von Medikamenten zu ermitteln und gegebenenfalls Behandlungsverfahren anzupassen, um sie effizienter zu machen. Weiterhin können Patientengruppen ermittelt werden, welche besonders gut auf ein bestimmtes Heilverfahren und/oder ein bestimmtes Medikament ansprechen und in der zentralen Datenbank eine entsprechende Information über die Wirksamkeit der entsprechenden Behandlungsressourcen für eine spezifische Patientengruppe hinterlegt werden.

In einer Ausgestaltung des erfindungsgemäßen Gesundheitsförderungssystems weist dieses eine Einrichtung zur Versorgung mit medizinischen Behandlungsressourcen auf. Dabei ist die zentrale Datenbank dazu eingerichtet, Informationen über verfügbare Behandlungsressourcen von der Einrichtung zur Versorgung mit medizinischen Behandlungsressourcen zu empfangen und zu speichern. Die Einrichtung zur Versorgung mit medizinischen Behandlungsressourcen kann zum Beispiel ein pharmazeutisches Unternehmen sein, das sich mit der Herstellung von pharmazeutischen Produkten oder der Bereitstellung von medizinischen Dienstleistungen befasst. Vorteilhaft werden bei dem erfindungsgemäßen Gesundheitsförderungssystem Daten von Herstellern und Dienstleistern hinsichtlich ihres Leistungsangebots mit in der zentralen Datenbank gespeichert, so dass sich die Betreuung einzelner Patienten und von Patientenkollektiven sehr vereinfacht.

Die Patientenbetreuungseinrichtung ihrerseits kann in einer Ausgestaltung des erfindungsgemäßen Gesundheitsförderungssystems einem Patienten und/oder seinem Betreuungspersonal die automatisiert ermittelten Informationen bezüglich seines Gesundheitszustands und spezifischer Behandlungsmöglichkeiten übermitteln. Dabei können diese Informationen auch spezifische Medikamente oder Behandlungsmöglichkeiten umfassen, die die zentrale Datenbank von der Einrichtung zur Versorgung mit medizinischen Behandlungsressourcen erhalten hat. Vorteilhaft können für die Patienten bereitgestellte Informationen auf die jeweils verfügbaren Ressourcen abgestimmt werden.

Die Volksgesundheitsverwaltungseinrichtung kann ferner dazu eingerichtet sein, die ermittelten statistischen Daten an die Einrichtung zur Versorgung mit medizinischen Behandlungsressourcen zu übermitteln. Vorteilhaft kann so die Einrichtung zur Versorgung mit medizinischen Behandlungsressourcen mit Informationen über die Wirksamkeit von Medikamenten in verschiedenen Teilen der Bevölkerung oder die Wirksamkeit von Behandlungsmethoden informiert werden. Mit dieser zusätzlichen Information können die entsprechenden Ressourcen verbessert und/oder zielgerichteter angewendet werden.

In einer Variante des erfindungsgemäßen Gesundheitsförderungssystems weist dieses eine medizintechnische bildgebende Einrichtung zum Erzeugen von medizinischen Bilddaten, welche Bildinformationen von Patienten umfassen, auf. Die medizinischen Bilddaten können zur Befundung für eine primäre Indikation an ein Ärzteteam übermittelt werden. Zusätzlich können die medizinischen Bilddaten an die Auswertungseinrichtung des erfindungsgemäßen Gesundheitsförderungssystems übermittelt werden und bezüglich sekundärer Indikationen ausgewertet werden. Vorteilhaft müssen für die zusätzlichen Indikationen nicht extra neue Bilddaten erzeugt werden, sondern es kann ein und derselbe Satz von Bilddaten für eine Vielzahl von medizinischen Fragestellungen genutzt werden.

In einer besonders bevorzugten Variante des erfindungsgemäßen Gesundheitsförderungssystem umfassen die Informationen über verfügbare Behandlungsressourcen Daten über verfügbare Medikamente und/oder Nahrungsergänzungsmittel und deren Indikationen, Daten über verfügbare Behandlungsmethoden und deren Indikationen und/oder Daten über verfügbare Behandlungsgeräte und deren Anwendung. Vorteilhaft können die zusätzlichen Informationen dazu genutzt werden, dem Patienten weiteren gesundheitlichen Nutzen aus den aufgenommenen medizinischen Bildern zu verschaffen.

In einer bevorzugten Ausgestaltung des erfindungsgemäßen Gesundheitsförderungssystem ist die Patientenbetreuungseinrichtung dazu eingerichtet, auf Basis der kombinierten medizinischen Daten eines Patienten die Informationen bezüglich seines Gesundheitszustands und spezifischer Behandlungsmöglichkeiten mit Hilfe eines auf einer Datenbasis trainierten automatisierten Auswertungsverfahrens zu ermitteln. Vorteilhaft kann für das Training des automatisierten Auswertungsverfahrens ein Teil der bereits in der zentralen Datenbank vorhandenen Datensätze als Datenbasis genutzt werden.

In einer Variante des erfindungsgemäßen Gesundheitsförderungssystems umfasst das trainierte automatisierte Auswertungsverfahren als Trainingsverfahren ein maschinelles Lernverfahren. Insbesondere kann das automatisierte Empfehlen von verfügbaren verschreibungspflichtigen oder frei verkäuflichen Medikamenten und/oder von verfügbaren Behandlungsmethoden durch maschinelles Lernen trainiert werden.

In vielen Gebieten werden Verfahren mit Lernalgorithmen, wie zum Beispiel "deep learning" oder "machine learning" angewandt. Eine große Anzahl von Datensätzen mit bekannten Informationen werden verwendet, um eine Software zu trainieren, die dann dazu in der Lage ist, vorher unbekannte Datensätze zu analysieren. Diese Verfahren werden aktuell breit angewandt für Aufgaben in der Bildanalyse, wie zum Beispiel Personenerkennung oder bei selbstfahrenden Fahrzeugen. Solche hochflexiblen automatisierten Lernverfahren sollen erfindungsgemäß auch für die Auswertung von medizinischen Bilddaten genutzt werden, um Zusatzinformationen für eine verbesserte Betreuung und eventuelle Behandlungs- oder Vorsorgemaßnahmen für Patienten zu gewinnen. Vorteilhaft kann mit einem solchen maschinellen Lernverfahren ein Auswertungsprozess flexibel und automatisiert an unterschiedliche Fragstellungen angepasst werden.

Bevorzugt umfassen die zusätzlichen medizinischen Daten, welche von dem Auswertungssystem des erfindungsgemäßen Gesundheitsförderungssystems ermittelt wurden, eine der folgenden Informationen:
- Knochenmineraldichte,
- Cholesterol-/Fettverteilung,
- Verteilung von Kalzifizierungen,
- Läsionen,
- Frakturen,
- erhöhter Blutdruck,
- Gefäßerweiterungen.

Vorteilhaft können mit Hilfe des erfindungsgemäßen Gesundheitsförderungssystems Bilddaten effizient als Datengrundlage für eine Vielzahl unterschiedlicher medizinischer Fragestellungen genutzt werden und gegebenenfalls zusätzliche Maßnahmen, wie zum Beispiel medizinische Behandlungen oder spezifische Überwachungsmaßnahmen durchgeführt werden.

Die von der Volksgesundheitsverwaltungseinrichtung ermittelten statistischen Daten können zum Beispiel umfassen:
- Informationen bezüglich der Verteilung eines spezifischen Gesundheitsparameters in der Bevölkerung,
- Informationen über die Inzidenz und/oder Prävalenz einer bestimmten Krankheit,
- Informationen bezüglich des Behandlungserfolgs mit einem bestimmten Medikament und/oder Heilverfahren.

Vorteilhaft können die statistischen Daten dazu genutzt werden, medizinische Ressourcen zielgerichteter und effizienter einzusetzen und deren Anwendung auf makroskopischer Ebene zu überwachen und gegebenenfalls anzupassen.

Die Erfindung wird im Folgenden unter Hinweis auf die beigefügten Figuren anhand von Ausführungsbeispielen noch einmal näher erläutert. Es zeigen:
FIG 1 ein Blockschaltbild, welches ein Gesundheitsförderungssystem gemäß einem Ausführungsbeispiel der Erfindung veranschaulicht,
FIG 2 ein Flussdiagramm, welches ein Gesundheitsbetreuungsverfahren gemäß einem Ausführungsbeispiel der Erfindung veranschaulicht.

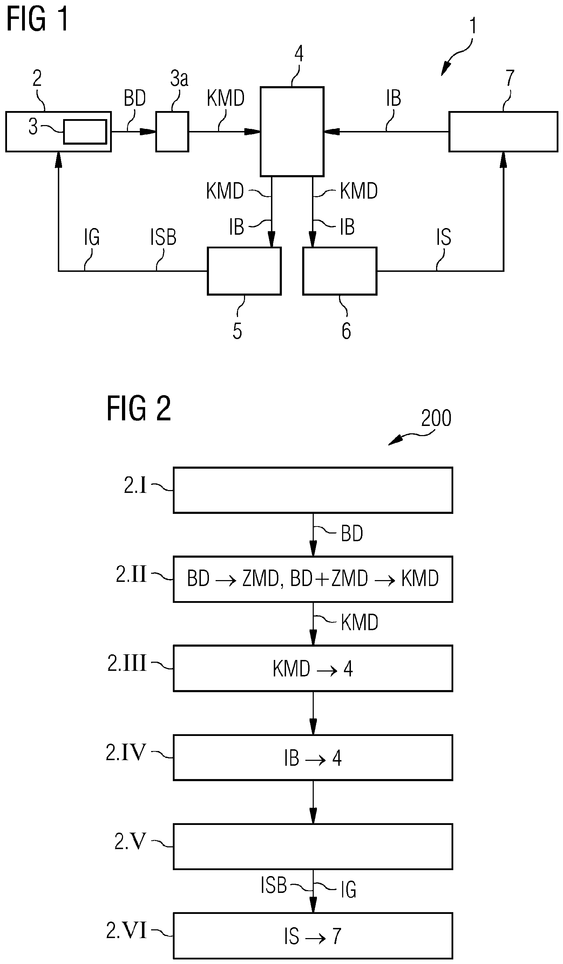

In FIG 1 ist ein Gesundheitsförderungssystem 1 gemäß einem Ausführungsbeispiel der Erfindung gezeigt, welches dazu dient, Patientendaten optimal zu nutzen. Das System 1 umfasst eine Patientenbehandlungseinrichtung 2, beispielsweise ein Hospital. Die Patientenbehandlungseinrichtung 2 weist eine medizintechnische bildgebende Einrichtung 3, beispielsweise ein CT-System, auf. Die von der medizintechnischen bildgebenden Einrichtung 3 von einem Patienten erzeugten Bilddaten BD werden zum Beispiel von einem Ärzteteam hinsichtlich einer primären Indikation ausgewertet. Beispielsweise werden Bilddaten von dem Thorax eines Patienten aufgenommen und nach Tumoren der Lunge untersucht. Zusätzlich werden die Bilddaten BD an eine Auswertungseinrichtung 3a übermittelt, die die Bilddaten BD, welche hinsichtlich einer primären Indikation ausgewertet wurden, hinsichtlich sekundärer medizinischer Indikationen auswertet und den sekundären medizinischen Indikationen zugeordnete zusätzliche medizinische Daten ZMD ermittelt.

Beispielsweise werden die Bilddaten BD des Thorax des Patienten zusätzlich hinsichtlich eines Calcium-Scores ausgewertet. Auf Basis der medizinischen Bilddaten BD und der zusätzlichen medizinischen Daten ZMD, in diesem Fall ein ermittelter Calcium Score, werden von der Auswertungseinrichtung 3a kombinierte medizinische Daten KMD gebildet. Diese kombinierten medizinischen Daten KMD werden dann an eine zentrale Datenbank 4 übermittelt.

Die zentrale Datenbank 4 speichert weiterhin auch Informationen IB über Medikamente und deren Anwendung, welche sie von einem pharmazeutischen Produzenten 7 erhält. Teil des Systems 1 ist auch eine Patientenbetreuungseinrichtung 5, welche auf Basis der in der zentralen Datenbank 4 gespeicherten Daten KMD, IB Informationen IG bezüglich des Gesundheitszustands eines Patienten und Informationen ISB bezüglich spezifischer Behandlungsmöglichkeiten ermittelt und diese Informationen IG, ISB an die Patientenbehandlungseinrichtung 2 weitergibt. Auf Basis dieser Informationen IG, ISB können nun dem Patienten für ihn geeignete Überwachungsprogramme oder Vorsorgemaßnahmen angeboten werden. Wie bereits erwähnt, können die in der zentralen Datenbank 4 gespeicherten kombinierten Daten KMD für einen Vergleich mit Bilddaten bzw. aus den Bilddaten extrahierten Merkmalen genutzt werden. Wird ein Datensatz KMD in der zentralen Datenbank 4 gefunden, dessen zugeordnete Merkmale denen der extrahierten Merkmale von Bilddaten eines neuen Patienten entsprechen, so können die in der zentralen Datenbank 4 abgespeicherten zusätzlichen Informationen über die Anwendung bestimmter Medikamente oder Heilverfahren auf den Datensatz des neuen Patienten übertragen werden und es können diesem Patient entsprechende Vorschläge für eine zusätzliche Behandlung gemacht werden.

Teil des Systems 1 ist auch ein Volksgesundheitsverwaltungssystem 6, welches dazu eingerichtet ist, auf Basis der in der zentralen Datenbank 4 gespeicherten Daten KMD, IB automatisiert statistische Daten IS betreffend den Gesundheitszustand bzw. die Verteilung bestimmter Gesundheitsparameter in einem Patientenkollektiv zu ermitteln. Weitere statistische Daten können auch Informationen betreffend Behandlungsergebnisse mit bestimmten Medikamenten oder Behandlungsmethoden sein. Diese statistischen Daten IS werden an die entsprechenden medizinischen Dienstleister oder Produzenten, in diesem Ausführungsbeispiel ein pharmazeutischer Produzent 7, übermittelt. Der pharmazeutische Produzent 7 nutzt die statistischen Daten dazu, seine Medikamente zu verbessern, bzw. Empfehlungen für den zielgerichteten Einsatz der Medikamente zu präzisieren.

In FIG 2 ist ein Flussdiagramm 200 gezeigt, welches ein Verfahren zur Betreuung von Patienten gemäß einem Ausführungsbeispiel der Erfindung veranschaulicht. Bei dem Schritt 2.I werden medizinische Bilddaten BD von Patienten erzeugt. Bei dem Schritt 2.II werden die medizinischen Bilddaten BD hinsichtlich sekundärer medizinischer Indikationen ausgewertet. D.h., es werden auf Basis der medizinischen Bilddaten BD Parameter ermittelt, welche Informationen enthalten, die hinsichtlich medizinischer Fragestellungen im Zusammenhang mit sekundären medizinischen Indikationen relevant sind. Dabei werden zusätzliche medizinische Daten ZMD erzeugt und diese zusammen mit den medizinischen Bilddaten BD zu kombinierten medizinischen Daten KMD zusammengefasst. Bei dem Schritt 2.III werden die kombinierten medizinischen Daten KMD in einer zentralen Datenbank 4 gespeichert.

Bei dem Schritt 2.IV werden zusätzlich Informationen IB über verfügbare Behandlungsressourcen von einer Einrichtung 7 zur Versorgung mit medizinischen Behandlungsressourcen in der zentralen Datenbank 4 gespeichert. Weiterhin erfolgt bei dem Schritt 2.V ein automatisiertes Ermitteln von Informationen IG, ISB bezüglich des Gesundheitszustands eines neuen Patienten und spezifischer Behandlungsmöglichkeiten auf Basis der in der zentralen Datenbank gespeicherten Daten KMD, IB durch eine Patientenbereuungseinrichtung 5, wobei ein bereits beschriebener Abgleich zwischen extrahierten Merkmalen, welche dem neuen Patienten zugeordnet sind und den entsprechenden Merkmalen in den in der zentralen Datenbank 4 gespeicherten Daten KMD vorgenommen wird. Diese Daten IG, ISB werden an eine Patientenbehandlungseinrichtung 2 weitergegeben, welche anhand dieser spezifischen Daten IG, ISB Überwachungsmaßnahmen oder Behandlungsvorgänge einleitet. Bei dem Schritt 2.VI werden statistische Daten IS betreffend den Gesundheitszustand von Patientenkollektiven und Statistiken über Behandlungsergebnisse auf Basis der in der zentralen Datenbank 4 gespeicherten Daten KMD, IB ermittelt und an die Einrichtung 7 zur Versorgung mit medizinischen Behandlungsressourcen übermittelt. Dort werden die Informationen IS dazu genutzt, Medikamente zu verbessern, bzw. Empfehlungen für den zielgerichteten Einsatz der Medikamente zu präzisieren.

Es wird abschließend noch einmal darauf hingewiesen, dass es sich bei den vorbeschriebenen Verfahren und Vorrichtungen lediglich um bevorzugte Ausführungsbeispiele der Erfindung handelt und dass die Erfindung vom Fachmann variiert werden kann, ohne den Bereich der Erfindung zu verlassen, soweit er durch die Ansprüche vorgegeben ist. Es wird der Vollständigkeit halber auch darauf hingewiesen, dass die Verwendung der unbestimmten Artikel "ein" bzw. "eine" nicht ausschließt, dass die betreffenden Merkmale auch mehrfach vorhanden sein können.

## Patentansprüche

1. Gesundheitsförderungssystem (1), aufweisend:
- eine Auswertungseinrichtung (3a), welche dazu eingerichtet ist,
- aus medizinischen Bilddaten (BD) von Patienten Merkmale hinsichtlich medizinischer Indikationen zu extrahieren und
- den medizinischen Bilddaten (BD) zugeordnete Medikamente und/oder Behandlungsverfahren zu ermitteln und auf Basis der extrahierten Merkmale und der ermittelten Medikamente und/oder Behandlungsverfahren kombinierte medizinische Daten (KMD) zu bilden,
- eine zentrale Datenbank (4), welche dazu eingerichtet ist, die kombinierten medizinischen Daten (KMD) von der Auswertungseinrichtung (3) zu empfangen und zu speichern und
- eine Patientenbetreuungseinrichtung (5), welche dazu eingerichtet ist, für einen neuen Patienten automatisiert Informationen (IG, ISB) bezüglich seines Gesundheitszustands und spezifischer Behandlungsmöglichkeiten auf Basis eines Vergleichs zwischen dem neuen Patienten zugeordneten medizinischen Bilddaten (BD) und den in der zentralen Datenbank (4) gespeicherten Daten (KMD, IB) zu ermitteln.

2. Gesundheitsförderungssystem nach Anspruch 1, ferner aufweisend eine Volksgesundheitsverwaltungseinrichtung (6), welche dazu eingerichtet ist, auf Basis der in der zentralen Datenbank (4) gespeicherten Daten (KMD, IB) automatisiert statistische Daten (IS) betreffend den Gesundheitszustand von Patientenkollektiven und Statistiken über Behandlungsergebnisse zu ermitteln.

3. Gesundheitsförderungssystem nach Anspruch 1 oder 2, aufweisend eine Einrichtung (7) zur Versorgung mit medizinischen Behandlungsressourcen, wobei die zentrale Datenbank (4) dazu eingerichtet ist, Informationen (IB) über verfügbare Behandlungsressourcen von der Einrichtung (7) zur Versorgung mit medizinischen Behandlungsressourcen zu empfangen und zu speichern.

4. Gesundheitsförderungssystem nach einem der vorstehenden Ansprüche, wobei die Patientenbetreuungseinrichtung (5) dazu eingerichtet ist, einem Patienten und/oder seinem Betreuungspersonal die ermittelten Informationen (IG, ISB) bezüglich seines Gesundheitszustands und spezifischer Behandlungsmöglichkeiten zu übermitteln.

5. Gesundheitsförderungssystem nach Anspruch 3 oder 4, wobei die Volksgesundheitsverwaltungseinrichtung (6) dazu eingerichtet ist, die ermittelten statistischen Daten (IS) an die Einrichtung (7) zur Versorgung mit medizinischen Behandlungsressourcen zu übermitteln.

6. Gesundheitsförderungssystem nach einem der vorstehenden Ansprüche 1 bis 5, aufweisend eine medizintechnische bildgebende Einrichtung (3) zum Erzeugen von medizinischen Bilddaten (BD), welche Bildinformationen (BI) von Patienten umfassen.

7. Gesundheitsförderungssystem nach einem der Ansprüche 3 bis 6, wobei die Informationen (IB) über verfügbare Behandlungsressourcen mindestens eine der folgenden Datenarten umfassen:
- Daten über verfügbare Medikamente und/oder Nahrungsergänzungsmittel und deren Indikationen,
- Daten über verfügbare Behandlungsmethoden und deren Indikationen
- Daten über verfügbare Behandlungsgeräte und deren Anwendung.

8. Gesundheitsförderungssystem nach einem der Ansprüche 1 bis 7, wobei die Patientenbetreuungseinrichtung (5) dazu eingerichtet ist, auf Basis der kombinierten medizinischen Daten (KMD) eines Patienten die Informationen (IG, ISB) bezüglich seines Gesundheitszustands und spezifischer Behandlungsmöglichkeiten mit Hilfe eines auf einer Datenbasis trainierten automatisierten Auswertungsverfahrens zu ermitteln.

9. Gesundheitsförderungssystem nach Anspruch 8, wobei das trainierte automatisierte Auswertungsverfahren als Trainingsverfahren ein maschinelles Lernverfahren umfasst.

10. Gesundheitsförderungssystem nach einem der vorstehen Ansprüche, wobei die zusätzlichen medizinischen Daten (ZMD) eine der folgenden Informationen umfassen:
- Knochenmineraldichte,
- Cholesterol-/Fettverteilung,
- Verteilung von Kalzifizierungen,
- Läsionen,
- Frakturen,
- erhöhter Blutdruck,
- Gefäßerweiterungen.

11. Gesundheitsförderungssystem nach einem der vorstehenden Ansprüche, wobei die statistischen Daten (IS) mindestens eine der folgenden Informationen umfassen:
- Informationen bezüglich der Verteilung eines spezifischen Gesundheitsparameters in der Bevölkerung,
- Informationen über die Inzidenz und/oder Prävalenz einer bestimmten Krankheit,
- Informationen bezüglich des Behandlungserfolgs mit einem bestimmten Medikament und/oder Heilverfahren.

12. Gesundheitsbetreuungsverfahren, aufweisend die Schritte:
- Erzeugen kombinierter medizinischer Daten (KMD) auf Basis von medizinischen Bilddaten (BD) von Patienten, wobei aus den medizinischen Bilddaten (BD) Merkmale hinsichtlich medizinischer Indikationen extrahiert werden und den medizinischen Bilddaten (BD) zugeordnete Medikamente und/oder Behandlungsverfahren ermittelt werden und auf Basis der extrahierten Merkmale und der medizinischen Bilddaten (BD) kombinierte medizinische Daten (KMD) gebildet werden,
- Speichern der kombinierten medizinischen Daten (KMD) in einer zentralen Datenbank (4),
- automatisiertes Ermitteln von Informationen (IG, ISB) bezüglich des Gesundheitszustands und spezifischer Behandlungsmöglichkeiten eines neuen Patienten auf Basis eines Vergleichs zwischen dem neuen Patienten zugeordneten medizinischen Bilddaten (BD) und den in der in der zentralen Datenbank (4) gespeicherten Daten (KMD, IB).
